# EUROPÄISCHE PATENTSCHRIFT

(11) **EP 2 961 436 B1**
(45) Veröffentlichungstag und Bekanntmachung des Hinweises auf die Patenterteilung: **27.05.2020**
(21) Anmeldenummer: 14707369.6
(22) Anmeldetag: 27.02.2014
(51) Int. Cl.: H05B 3/82, H05B 6/80, A61L 2/07, A61L 2/24, A47L 15/42

(54) **REINIGUNGSVORRICHTUNG ZUR REINIGUNG VON BEHÄLTERN FÜR MENSCHLICHE AUSSCHEIDUNGEN**
CLEANING DEVICE FOR CLEANING CONTAINERS FOR HUMAN EXCRETIONS
DISPOSITIF DE NETTOYAGE DESTINÉ À NETTOYER DES CONTENANTS À DÉJECTIONS HUMAINES

(30) Priorität: 28.02.2013 DE 102013203342
(43) Veröffentlichungstag der Anmeldung: 06.01.2016
(73) Patentinhaber: MEIKO Maschinenbau GmbH & Co. KG, 77652 Offenburg (DE)
(72) Erfinder: HOFER, Martin, Ebersweier 77770 (DE); WIEGAND, Ingo, Bühlertal 77830 (DE)
(74) Vertreter: Altmann Stößel Dick Patentanwälte PartG mbB
(86) Internationale Anmeldenummer: PCT/EP2014/053814
(87) Internationale Veröffentlichungsnummer: WO 2014/131827

(56) Entgegenhaltungen:
- EP-A1- 1 300 630
- EP-A1- 2 060 275
- DE-A1- 19 838 180
- DE-A1-102007 025 263
- DE-A1-102007 036 769

## Beschreibung

### Gebiet der Erfindung

Die Erfindung betrifft eine Reinigungsvorrichtung sowie ein Verfahren zur Reinigung mindestens eines Behälters für menschliche Ausscheidungen. Derartige Reinigungsvorrichtungen und Verfahren werden beispielsweise im Krankenhaus- oder Pflegebereich eingesetzt, um Behälter wie beispielsweise Steckbecken, Urinflaschen, Nierenschalen, Waschschüsseln oder andere Behälter zu reinigen, welche zur Aufnahme menschlicher oder tierischer Ausscheidungen geeignet sind.

### Stand der Technik

Aus dem Stand der Technik sind eine Vielzahl von Reinigungsvorrichtungen und Reinigungsverfahren zur Behandlung von Behältern für menschliche Ausscheidungen bekannt. Die zu reinigenden Behälter können größere Flüssigkeitsmengen oder Mengen an Feststoffabfällen enthalten, welche üblicherweise während der Reinigung entsorgt werden müssen. Zudem können derartige Behälter infektiöse Abfälle enthalten oder anderweitig kontaminiert sein, sodass, neben der Entleerung, auch üblicherweise eine Desinfektion erforderlich ist. Neben den genannten Behältern für menschliche Ausscheidungen sind diese Reinigungsvorrichtungen grundsätzlich auch zur Reinigung anderer medizinischer Gegenstände geeignet, wie sie beispielsweise in Krankenhäusern oder Altenpflegeeinrichtungen eingesetzt werden. Üblicherweise besteht das Reinigungsgut jedoch aus Urinflaschen, Steckbecken, Nierenschalen, Waschschüsseln oder ähnlichen Behältern, deren Reinigung die Entsorgung größerer Abfallmengen mit sich bringen kann.

Diese Reinigungsvorrichtungen weisen häufig einen Fluidtank und einen Dampferzeuger auf, wobei verschiedene Bauarten von Fluidtanks und Dampferzeugern bekannt sind. In DE 198 31 950 C2 wird eine Maschine zum Reinigen und Desinfizieren von Pflegegeschirr beschrieben. Das Pflegegeschirr wird beispielsweise in Krankenhäusern eingesetzt. Um die Aufheizzeiten eines Dampferzeugers, eine Verkeimungsgefahr und ein Wasservolumen zu verringern und um eine Arbeitsgeschwindigkeit zu erhöhen, wird eine Heizeinrichtung eines Verdampfers der Maschine in einen Wasserkasten integriert.

Die zur Desinfektion zur Verfügung stehende Flüssigkeitsmenge ist in der Regel abhängig von der Bauweise des Dampferzeugers. Beispielsweise beschreibt EP 0 874 580 B1 einen Dampferzeuger mit Heizelement, der in einem Schacht des Wassertanks integriert ist. Für den Desinfektionsschritt verbleibt nach dem Waschprozess Wasser bis zu einem bestimmten Niveau, welches einer Oberkante des Schachts entspricht, im Tank und wird verdampft. Während des Desinfektionsschritts wird kein Frischwasser in den Wassertank nachgefüllt. Die Füllmenge des Dampferzeugers ist bestimmt durch die Schachtgröße. DE 29 00 957 A1 beschreibt ebenfalls einen Dampferzeuger mit einer fest vorgegebenen Füllmenge des Dampferzeugertanks. Dieser röhrenförmige Dampferzeuger wird solange mit Frischwasser aus dem Wassertank über eine Dosierpumpe befüllt bis eine bestimmte Füllmenge erreicht ist. Dann wird mit einem Niveauausschalter eine Dosierpumpe abgeschaltet.

Eine baulich fest vorgegebene Füllmenge im Dampferzeuger ist nachteilig, da so einerseits der Dampferzeugertank häufig mit mehr Fluid gefüllt ist als für den Desinfektionsschritt benötigt wird. Unnötig lange Aufheizzeiten und ein hoher Stromverbrauch sind die Folge. Zudem werden häufig dem Dampferzeuger chemische Zusatzstoffe zugegeben, beispielsweise gegen die Bildung von Kalkbelägen, wobei die Menge der Zusatzstoffe abhängig sein kann von der Füllmenge im Tank des Dampferzeugers. Eine zu hohe Füllmenge des Dampferzeugertanks führt dann in vielen Fällen zu einem hohen Verbrauch an chemischen Zusatzstoffen.

Eine baulich fest vorgegebene Füllmenge im Dampferzeuger ist zudem nachteilig, da die meisten Reinigungsvorrichtungen für niedrige und mittlere Desinfektionsleistungen konstruiert werden. Wird eine höhere Füllmenge als die maximale baulich fest vorgegebene Füllmenge des Dampferzeugertanks benötigt, beispielsweise im Seuchenfall, ist dies kurzfristig nicht möglich, so dass dann in der Regel ein Umbau der Reinigungsvorrichtung erforderlich ist.

Eine Reinigungsvorrichtung mit variabler Füllmenge wird in DE 195 09 877 C2 offenbart. Darin ist ein Dampferzeuger an seiner Bodenseite hydraulisch mit einem Wasservorratstank verbunden, und nach dem Prinzip kommunizierender Gefäße ist der Flüssigkeitsstand im Dampferzeugertank immer so hoch wie im Wassertank. Der Wassertank dient als Frischwasserreservoir für den Dampferzeuger, wobei in den Wassertank ständig Wasser nachgefüllt werden kann, so dass die Dampfmenge nicht beschränkt ist. Nachteilig ist, dass, bedingt durch das Prinzip kommunizierender Gefäße, das Niveau der Flüssigkeit im Dampferzeuger abhängig vom Flüssigkeitsstand in dem Fluidtank ist. Während des Desinfektionsschritts wird der Flüssigkeitsstand im Dampferzeuger sinken, so dass ständig Wasser aus dem Fluidtank in den Dampferzeuger nachgefüllt wird um den Flüssigkeitsstand auszugleichen. Dadurch muss eine größere Wassermenge geheizt werden als für die Desinfektion benötigt wird. Ein weiterer Nachteil bei einigen Ausführungsformen von Reinigungsvorrichtungen ist der sehr hohe apparative Aufwand.

In DE 10 2007 025 263 A1 wird ein Reinigungsgerät zur Reinigung von Reinigungsgut beschrieben. Die Reinigungsvorrichtung weist einen Mikrowellen-Dampferzeuger auf, welcher mit kleinsten zu verdampfenden Flüssigkeitsmengen auskommt. Zudem wird beschrieben, dass in einer Verdampfungskammer ein Niveausensor aufgenommen sein kann, um ein Überfüllen der Verdampfungskammer zu verhindern.

DE 10 2007 025 245 A1 offenbart eine Vorrichtung zur Erwärmung eines Tankinhaltes eines Tanks, beispielsweise eines Reinigungs- und Desinfektionsautomaten. Die Vorrichtung weist eine Wasser/Dampf-Einheit mit einem Dampferzeuger auf. Am Boden des Dampferzeugers kann eine Vakuum-Laufzeitröhre vorgesehen sein, über welche Mikrowellen in den im Dampferzeuger bevorrateten Wasservorrat eingetragen werden und diesen erwärmen.

DE 10 2007 021 245 beschreibt ein Verfahren zur Reinigung von Spülgut mit größerem Flüssigkeitsanfall, insbesondere in einem Steckbeckenspüler. Der Reinigungsprozess des Steckbeckenspülers kann durch eine Steuerung überwacht werden. Die Steuerung kann eine Information über einen Zielkeim empfangen, in ein entsprechendes Zielthermoäquivalent umwandeln und an einzelne Reinigungsvorrichtungen weiterleiten. In den Reinigungsvorrichtungen können entsprechend dieser Vorgaben dann geeignete Prozessparameter ausgewählt werden.

Trotz der mit diesen bekannten Vorrichtungen und Verfahren erzielten Vorteile bleiben nach wie vor eine Vielzahl an technischen Herausforderungen bestehen. So besteht nach wie vor ein Bedarf an einer weiteren Senkung des Verbrauchs an Ressourcen und Energie. Gleichzeitig soll jedoch jederzeit eine ausreichende Hygienisierungswirkung des Reinigungsverfahrens sichergestellt sein.

### Aufgabe

Es ist daher Aufgabe der vorliegenden Erfindung, eine Reinigungsvorrichtung und ein Verfahren zur Reinigung eines Behälters für menschliche Ausscheidungen bereitzustellen, welche die Nachteile bekannter Reinigungsvorrichtungen und Verfahren zumindest weitgehend vermeiden. Insbesondere soll eine bedarfsangepasste Befüllung eines Dampferzeugertanks möglich sein, wobei vorzugsweise kurze Aufheizzeiten und ein geringer Verbrauch von Ressourcen wie Wasser, Strom und chemischen Zusatzstoffen erreicht werden sollen.

### Beschreibung der Erfindung

Diese Aufgabe wird gelöst durch eine Reinigungsvorrichtung und ein Verfahren mit den Merkmalen der unabhängigen Patentansprüche. Vorteilhafte Weiterbildungen der Erfindung, welche einzeln oder in beliebiger Kombination realisierbar sind, sind in den abhängigen Patentansprüchen dargestellt.

Die beschriebene Reinigungsvorrichtung ist eingerichtet, um ein erfindungsgemäßes Verfahren durchzuführen. Zu diesem Zweck weist die Reinigungsvorrichtung eine entsprechende Steuerung auf. Umgekehrt wird das Verfahren unter Verwendung der erfindungsgemäßen Reinigungsvorrichtung durchgeführt. Dementsprechend kann für die Beschreibung möglicher Details der Reinigungsvorrichtung auf die Beschreibung optionaler Details des Verfahrens verwiesen werden und umgekehrt.

Weiterhin wird darauf hingewiesen, dass die Begriffe "aufweisen" und "umfassen" sowie entsprechende grammatikalische Abwandlungen dieser Verben im Folgenden im nichtausschließlichen Sinne verwendet werden. So beschreibt der Sachverhalt "A weist B" auf oder der Sachverhalt "A umfasst B" sowohl die Möglichkeit, dass A ausschließlich aus B besteht oder auch, alternativ, den Sachverhalt, das A neben B, eine oder mehrere weitere Komponenten und/oder Bestandteile beinhaltet.

In einem ersten Aspekt wird eine Reinigungsvorrichtung zur Reinigung mindestens eines Behälters für menschliche Ausscheidungen vorgeschlagen. Beispielsweise kann diese Reinigungsvorrichtung ein Reinigungs- und Desinfektionsgerät sein. Die Reinigungsvorrichtung kann beispielsweise gemäß dem oben beschriebenen Stand der Technik ausgestaltet sein, mit den nachfolgend beschriebenen zusätzlichen erfindungsgemäßen Merkmalen. Auch eine andere Ausgestaltung ist jedoch möglich.

Unter einem Behälter zur Aufnahme menschlicher Ausscheidungen ist insbesondere ein Behälter aus der Gruppe von Urinflaschen, Steckbecken, Nierenschalen, Waschschüsseln oder ähnlichen Behältern, wie sie beispielsweise in Krankenhäusern oder Pflegeeinrichtungen verwendet werden, zu verstehen, deren Reinigung die Entsorgung größerer Abfallmengen mit sich bringen kann. Beispielsweise kann der Behälter eingerichtet sein, um Ausscheidungen in einer Menge von mindestens 100 ml, insbesondere in einer Menge von mindestens 200 ml oder sogar mindestens 500 ml, aufzunehmen.

Der Behälter kann mindestens eine Behälterwand aufweisen, welche starr oder auch verformbar, insbesondere flexibel, ausgestaltet sein kann. So kann der Behälter beispielsweise ein Gefäß mit einer starren Behälterwand, beispielsweise aus einem oder mehreren der Materialien Glas, Kunststoff, Keramik und Metall, aufweisen. Alternativ oder zusätzlich kann der Behälter auch mindestens eine verformbare Behälterwand, beispielsweise mindestens einen Folienbeutel, insbesondere einen Folienbeutel aus einem Kunststoffmaterial, aufweisen. So kann der Behälter für menschliche Ausscheidungen beispielsweise auch ganz oder teilweise als zunächst geschlossenes Behältnis in Gestalt eines Folienbeutels ausgebildet sein.

Der Behälter kann mindestens einen Aufnahmebereich für menschliche Ausscheidungen aufweisen, zum Beispiel mindestens einen Hohlraum. Weiter kann der Behälter mindestens eine Öffnung aufweisen. Diese Öffnung kann von vorneherein vorhanden sein und/oder kann auch zu einem späteren Zeitpunkt geschaffen werden. Weiterhin kann auch während und/oder vor der Behandlung mindestens eine Öffnung geschaffen werden, beispielsweise durch ein mechanisches Öffnen des Behälters und/oder durch ein Aufschneiden und/oder Aufreißen des Behälters. Weiterhin kann die Öffnung eingerichtet sein, um reversibel oder irreversibel geöffnet und/oder verschlossen zu werden.

Die Reinigungsvorrichtung umfasst mindestens eine Reinigungskammer zur Aufnahme des mindestens einen Behälters. Eine Reinigungskammer im Sinne der vorliegenden Erfindung ist eine vollständig oder teilweise geschlossene Kammer zur Aufnahme des Behälters. Die Reinigungskammer kann insbesondere mindestens eine Öffnung aufweisen, durch welche der mindestens eine Behälter in einen Innenraum der Kammer eingebracht werden kann. Die Öffnung kann insbesondere verschließbar ausgestaltet sein, beispielsweise mittels mindestens einer Tür und/oder Klappe.

Die Reinigungsvorrichtung kann mindestens eine Halterung aufweisen. Unter einer Halterung ist allgemein eine Vorrichtung zu verstehen welche eingerichtet ist, um den Behälter aufzunehmen und relativ zur Halterung zu fixieren. Beispielsweise kann diese Halterung mindestens eine Schiene umfassen, in welche der Behälter eingeschoben werden kann. Alternativ oder zusätzlich kann die Halterung auch einen oder mehrere Bügel und/oder einen oder mehrere Hohlräume umfassen, in welche der Behälter eingeschoben und/oder eingebracht werden kann. Dabei kann die Reinigungsvorrichtung eine feste Halterung aufweisen oder auch eine austauschbare Halterung, beispielsweise indem eine Art der Halterung auf den Typ der Behälters angepasst werden kann.

Die Reinigungsvorrichtung kann eingerichtet sein, um den Behälter in einen Abfluss zu entleeren. Das Entleeren kann beispielsweise automatisch erfolgen, beispielsweise vor und/oder während einem Reinigungsprogramm. Beispielsweise kann die Entleerung beim Schließen einer Tür erfolgen, insbesondere falls eine Halterung für den mindestens einen Behälter mit der Tür verbunden ist.

Zur Realisierung einer Entleerung kann die Reinigungskammer beispielsweise im Bodenbereich zumindest teilweise trichterförmig und/oder geneigt auf den Abfluss zulaufen. Insbesondere kann der Abfluss einen Geruchsverschluss aufweisen. Unter einem Geruchsverschluss ist eine Vorrichtung zu verstehen, mit der Gase aus mindestens einem mit dem Abfluss verbundenen Abflussrohr von dem Inneren der Reinigungskammer ferngehalten werden können. Beispielsweise kann der Geruchsverschluss mindestens einen Siphonbogen aufweisen. Der Siphonbogen kann beispielsweise eine Flüssigkeitsmenge als Geruchsverschluss aufweisen. Zudem kann die Reinigungsvorrichtung vorzugsweise mindestens einen Bypass aufweisen, um Gas und Dampf aus der Reinigungskammer und der Umgehung des Geruchsverschlusses, beispielsweise unter Umgehung des Siphonbogens, in den Abfluss zu leiten, vorzugsweise unter Druck. Dazu umfasst der Bypass beispielsweise eine oder mehrere Rohrleitungen, die die Reinigungskammer mit dem Abfluss unter Umgehung des Geruchsverschlusses verbinden, sowie optional mindestens ein Rückschlagventil und/oder mindestens eine andere Art von Ventil, das ein Eindringen von Gasen aus dem Abfluss und/oder einem mit dem Abfluss verbundenen Abflussrohr durch den Bypass in die Reinigungskammer ganz oder teilweise verhindert.

Die Reinigungsvorrichtung weist mindestens eine Steuerung auf, welche eingerichtet ist, um mindestens ein Reinigungsprogramm durchzuführen. Das Reinigungsprogramm umfasst mindestens einen Waschschritt und mindestens einen Desinfektionsschritt. In dem mindestens einen Waschschritt wird der mindestens eine Behälter mit mindestens einem Reinigungsfluid beaufschlagt. In dem mindestens einen Desinfektionsschritt wird der Behälter mit Heißdampf beaufschlagt. Dabei kann insbesondere der mindestens eine Waschschritt vor dem mindestens einen Desinfektionsschritt durchgeführt werden. Es ist jedoch grundsätzlich auch ein komplexeres Verfahren denkbar. Jeder der genannten Verfahrensschritte kann einfach oder auch mehrfach durchgeführt werden.

Die Reinigungskammer weist mindestens eine Fluidvorrichtung zur Beaufschlagung des Behälters mit dem mindestens einem Reinigungsfluid auf. Im Rahmen der vorliegenden Erfindung ist unter einer Fluidvorrichtung eine Vorrichtung zu verstehen, mit der eine Flüssigkeit direkt auf den Behälter aufgebracht werden kann, beispielsweise durch Besprühen, Betropfen oder Bestrahlen oder eine Kombination der genannten Beaufschlagungen und/oder auf andere Weise. Alternativ oder zusätzlich kann die Fluidvorrichtung auch eingerichtet sein, um die Beaufschlagung derart vorzunehmen, dass ein vollständiges oder teilweises Befüllen der Reinigungskammer mit Reinigungsfluid erfolgt.

Die Fluidvorrichtung kann zur Beaufschlagung des Behälters mit Reinigungsfluid insbesondere mindestens eine Düse aufweisen. Diese Düse kann insbesondere mindestens eine Öffnung aufweisen, beispielsweise mindestens eine Sprühöffnung oder eine Mehrzahl Sprühöffnungen, durch welche das Reinigungsfluid in die Reinigungskammer eintreten kann. Weiterhin kann durch dieselbe mindestens eine optionale Düse der Fluidvorrichtung und/oder durch mindestens eine andere Art von Öffnung Heißdampf in die Reinigungskammer eingelassen werden, so dass die Fluidvorrichtung optional auch zur Beaufschlagung des Behälters mit Heißdampf verwendet werden kann.

Unter einem Reinigungsfluid kann insbesondere eine beliebige Flüssigkeit und/oder ein beliebiges Gas verstanden werden, welche eine reinigende Wirkung auf den Behälter aufweisen. Beispielsweise kann das Reinigungsfluid Wasser oder Wasser mit mindestens einem optionalen Zusatzstoff sein. Der mindestens eine Zusatzstoff kann beispielsweise mindestens ein chemisches Desinfektionsmittel umfassen und/oder mindestens ein Reinigungsmittel, beispielsweise ein Reinigerkonzentrat. Vorzugsweise liegt das Reinigungsfluid in flüssiger Form vor. Allgemein können ein oder mehrere Reinigungsfluide verwendet werden, wobei beispielsweise auch in unterschiedlichen Verfahrensschritten unterschiedliche Arten von Reinigungsfluiden eingesetzt werden können.

Die Reinigungsvorrichtung kann zur Speicherung von Reinigungsfluid insbesondere mindestens einen Fluidtank aufweisen. Dieser Fluidtank kann beispielsweise ein Volumen des Reinigungsfluids aufnehmen, beispielsweise ein Wasservolumen. Der Fluidtank kann beispielsweise mit einem Frischwasserzulauf verbunden sein. Weiterhin kann der Fluidtank beispielsweise mit mindestens einer Dosiervorrichtung verbunden sein, beispielsweise um einen oder mehrere Zusatzstoffe in den Fluidtank zu dosieren, beispielsweise ein oder mehrere Desinfektionsmittel und/oder ein oder mehrere Reinigerkonzentrate. Der Fluidtank kann beispielsweise über mindestens eine Fluidleitung, beispielsweise mindestens eine Rohrleitung, mit der Reinigungskammer verbunden sein. Die Reinigungsvorrichtung kann weiterhin beispielsweise mindestens eine Waschpumpe aufweisen, um das Reinigungsfluids in die Reinigungskammer zu pumpen, beispielsweise zu der mindestens einen Düse.

In dem mindestens einen Desinfektionsschritt wird der Behälter, wie oben ausgeführt, mit Heißdampf beaufschlagt. Beispielsweise kann es sich bei diesem Heißdampf um Wasserdampf handeln, beispielsweise Wasserdampf mit einer Temperatur von mindestens 80 °C, vorzugsweise mindestens 90 °C oder sogar mindestens 100 °C handeln. Auch Dampfgemische sind jedoch grundsätzlich möglich, beispielsweise Dampfgemische aus Wasserdampf, vorzugsweise mit der genannten Temperatur, und mindestens einer zusätzlichen Gaskomponente, beispielsweise einem desinfizierenden Gas.

Die Reinigungsvorrichtung umfasst zur Erzeugung von Dampf einen Dampferzeuger. Dieser kann ein in den Fluidtank integriertes oder ein separates Bauteil sein. In ersterem Fall kann die Reinigungsvorrichtung beispielsweise mindestens eine Fluideinheit umfassen, welche gleichzeitig mindestens einen Fluidtank und den Dampferzeuger umfasst, wobei die Fluideinheit mit der Reinigungskammer über mindestens eine Rohrleitung verbunden sein kann. In letzterem Fall kann der Dampferzeuger getrennt von dem Fluidtank ausgebildet sein und mit diesem beispielsweise fluidisch verbunden sein.

Allgemein kann der Dampferzeuger gemeinsam mit der Reinigungskammer in eine Einheit der Reinigungsvorrichtung integriert sein oder kann auch ganz oder teilweise separat von der Reinigungskammer angeordnet sein und mit der Reinigungskammer beispielsweise über eine oder mehrere Rohrleitungen verbunden sein. Die mindestens eine Rohrleitung kann beispielsweise auch in dem Waschschritt zum Befördern des Reinigungsfluids genutzt werden, oder es kann mindestens eine separate Rohrleitung ausschließlich zum Befördern von Heißdampf vorgesehen sein.

Besonders vorteilhaft ist eine Bauart einer Reinigungsvorrichtung, bei der der Dampferzeuger und der Fluidtank in einer gemeinsamen Fluideinheit integriert sind, wobei der Dampferzeugerbereich vorzugsweise durch eine Trennwand vom Fluidtank getrennt ist. Vorzugsweise ist in der Trennwand mindestens eine fluidische Verbindung zwischen dem Fluidtank und dem Dampferzeuger vorgesehen, beispielsweise mindestens eine fluidische Verbindung ausgewählt aus der Gruppe bestehend aus: einer Öffnung in der Trennwand; einer den Fluidtank und den Dampferzeuger verbindenden Leitung; einem Durchlass in der Trennwand; einem Überlauf. Beispielsweise kann die Trennwand derart ausgebildet sein, das die Trennwand nicht bis zu einem Deckel des Fluidtanks reicht, sondern vorzugsweise eine Verbindung im oberen Bereich des Fluidtanks zwischen einem Fluidbereich und einem Dampfbereich frei bleibt.

Allgemein ist die Reinigungsvorrichtung derart ausgebildet, das der Tank des Dampferzeugers über den Fluidtank befüllbar ist, also dass Reinigungsfluid oder ein Teil des Reinigungsfluids, insbesondere Wasser, aus dem Fluidtank in den Tank des Dampferzeugers gefüllt werden kann.

Die Reinigungsvorrichtung ist so eingerichtet, dass der Tank des Dampferzeugers in mindestens einem Befüllschritt aus dem Fluidtank befüllt wird. Beispielsweise kann die Befüllung über mindestens einen Überlauf erfolgen. Der Befüllschritt kann insbesondere ganz oder teilweise vor dem Waschschritt durchgeführt werden. Der Tank des Dampferzeugers ist dabei vorzugsweise ganz oder teilweise unterhalb des Überlaufs angeordnet. Der Überlauf umfasst vorzugsweise mindestens eine Überlaufkante, die beispielsweise ein maximales Niveau zum Füllen des Tanks des Dampferzeugers festlegen kann.

Der im Desinfektionsschritt erzeugte Heißdampf kann zur Beaufschlagung des Behälters in die Reinigungskammer geleitet werden, beispielsweise über mindestens eine Rohrleitung. Wie oben ausgeführt, kann dabei beispielsweise mindestens eine Rohrleitung verwendet werden, welche auch im Waschschritt zum Leiten des Reinigungsfluids verwendet wird, und/oder es kann mindestens eine separate Rohrleitung verwendet werden. Wird dieselbe Rohrleitung verwendet, so bietet dies beispielsweise den Vorteil, dass die Rohrleitung für das Reinigungsfluid durch den Heißdampf desinfiziert werden kann.

Die Reinigungsvorrichtung kann so eingerichtet sein, dass der benötigte Füllstand des Fluids in dem Fluidtank für den Waschschritt, auch als Waschniveau bezeichnet, unterhalb der Überlaufkante des Fluidtanks oder höchstens auf gleicher Höhe wie die Überlaufkante liegt. Diese Bauart der Reinigungsvorrichtung ist besonders vorteilhaft, da Waschschritte unabhängig von einer Befüllung des Tanks des Dampferzeugers durchgeführt werden können.

Zur Durchführung des Reinigungsprogramms weist die Reinigungsvorrichtung mindestens eine Steuerung auf. Die Steuerung kann zentral oder dezentral sein. Die Steuerung kann beispielsweise mindestens eine Datenverarbeitungsvorrichtung umfassen, beispielsweise mindestens einen Prozessor, insbesondere mindestens einen Mikrokontroller. Weiter kann die Steuerung eine oder mehrere Schnittstellen aufweisen, beispielsweise mindestens eine Benutzerschnittstelle und/oder mindestens eine elektronische Schnittstelle. Beispielsweise kann die mindestens eine Schnittstelle eine Tastatur und/oder ein Bediendisplay umfassen, über das ein Benutzer ein oder mehrere Parameter, wie beispielsweise eine vorgegebene Reihenfolge und Zeitdauer von Waschschritten und Desinfektionsschritten, des Reinigungsprogramms einstellen kann. Eine elektronische Schnittstelle kann beispielsweise eine Verbindung zu einer anderen Maschine, beispielsweise einem Computer oder Computernetzwerk, bereitstellen, wobei beispielsweise Informationen und/oder Befehle zwischen der Maschine und der Reinigungsvorrichtung oder umgekehrt ausgetauscht werden können. Beispielsweise können auf diese Weise Informationen über zu erwartende Verschmutzungen und/oder Keime an die Reinigungsvorrichtung übermittelt werden. Die Schnittstelle kann unidirektional oder bidirektional sein.

Die Steuerung ist eingerichtet, um für den Desinfektionsschritt aus einer Mehrzahl möglicher Flüssigkeitsmengen eine Flüssigkeitsmenge auszuwählen und den Tank des Dampferzeugers vor Durchführung des Desinfektionsschritts mit der ausgewählten Flüssigkeitsmenge zu befüllen. Beispielsweise kann die Mehrzahl möglicher Flüssigkeitsmengen in einem Datenspeicher der Steuerung hinterlegt sein oder auf andere Weise vorgegeben sein. Insbesondere kann die Anzahl möglicher Flüssigkeitsmengen beschränkt sein, so dass beispielsweise 2 bis 100, insbesondere 3 bis 20 möglicher Flüssigkeitsmengen auswählbar sein können. Alternativ ist jedoch auch eine stufenlose Auswahl möglicher Flüssigkeitsmengen realisierbar.

Für den Desinfektionsschritt kann das Desinfektionsergebnis vorzugsweise bedarfsgerecht mittels der Steuerung durch ein vorgebbares Desinfektionsergebnis ausgewählt werden. Dazu kann die Steuerung aus einer Mehrzahl möglicher Flüssigkeitsmengen beispielsweise eine Flüssigkeitsmenge auswählen und füllt den Tank des Dampferzeugers auf einen bedarfsgerechten Füllstand. Die Flüssigkeitsmenge, mit der der Dampferzeuger befüllt wird, kann die Dampfmenge bestimmen, die während des Desinfektionsschritts erzeugt werden kann.

Der Dampfbedarf im Desinfektionsschritt korreliert in der Regel mit dem erwünschten Desinfektionsergebnis, welches beispielsweise in Form von Thermoäquivalenten definiert werden kann. Allgemein werden Thermoäquivalente beispielsweise nach EN ISO 15883 oder dem NSF 3-Standard, beispielsweise ausgewählt aus A0-Werten oder H.U.E.-Werten, verwendet.

Die Steuerung kann insbesondere eingerichtet sein, um die Flüssigkeitsmenge entsprechend einem vorgebbaren Desinfektionsergebnis auszuwählen. Unter einem Desinfektionsergebnis kann beispielsweise eine messbare Keimreduktion oder Keimabtötung verstanden werden, welche beispielsweise mittels Abklatschuntersuchungen festgestellt werden kann. Diesbezüglich kann beispielsweise auf EN ISO 15883 und/oder NSF3 verwiesen werden. Alternativ oder zusätzlich kann auch beispielsweise mindestens ein Zielerreger vorgegeben werden, beispielsweise über die oben genannte Schnittstelle, beispielsweise durch einen Benutzer und/oder durch einen Computer oder ein Computernetzwerk. Weiterhin kann beispielsweise auf DE 10 2004 056 052 A1 und/oder DE 10 2007 025 263 A1 sowie den dort beschriebenen Stand der Technik verwiesen werden.

Das Desinfektionsergebnis kann insbesondere in Form einer Menge an Thermoäquivalenten definierbar sein. Auch diesbezüglich kann beispielsweise auf DE 10 2004 056 052 A1 und/oder DE 10 2007 025 263 A1 sowie den dort beschriebenen Stand der Technik verwiesen werden. Die Thermoäquivalente können insbesondere vorgegeben sein nach einem Standard ausgewählt aus EN ISO 15883 oder NSF3. Das Desinfektionsergebnis kann insbesondere in Form von Thermoäquivalenten definierbar sein, ausgewählt aus A0-Werten oder H.U.E.-Werten.

Die Reinigungsvorrichtung, insbesondere die Steuerung, kann vorzugsweise eingerichtet sein, um ein tatsächliches Desinfektionsergebnis einer Desinfektion des Behälters während des Desinfektionsschritts zu überwachen. Zur Überwachung des tatsächlichen Desinfektionsergebnisses können insbesondere Temperaturmessungen über einen Zeitraum hinweg durchgeführt werden. So kann die Reinigungsvorrichtung, insbesondere die Steuerung, eingerichtet sein, um einen zeitlichen Temperaturverlauf in der Reinigungskammer zu erfassen und das tatsächliche Desinfektionsergebnis aus dem zeitlichen Temperaturverlauf zu bestimmen, insbesondere durch Berechnung einer tatsächlichen Menge an Thermoäquivalenten, mit denen der Behälter beaufschlagt wird. Dabei können beispielsweise sämtliche Zeiträume während des Desinfektionsschritts erfasst werden oder lediglich Zeiträume, innerhalb derer die Temperatur, mit welcher der Behälter beaufschlagt wird, oberhalb einer oder mehrerer Schwelltemperaturen liegt. So können beispielsweise lediglich Zeiträume erfasst werden, innerhalb derer der Behälter mit mindestens einer Mindesttemperatur beaufschlagt wird. Aus der Temperatur und der jeweiligen Zeit der Beaufschlagung können dann in üblicher Weise, wie beispielsweise in DE 10 2004 056 052 A1 und/oder DE 10 2007 025 263 A1 sowie in dem dort genannten Stand der Technik beschrieben, Thermoäquivalente berechnet werden, welche ein tatsächliches Desinfektionsergebnis beschreiben. Dieses tatsächliche Desinfektionsergebnis kann beispielsweise mit dem mindestens einen vorgegebenen Desinfektion Ergebnis verglichen werden. Beispielsweise kann der Desinfektionsschritt abgebrochen werden, wenn das tatsächliche Desinfektionsergebnis das vorgegebene Desinfektionsergebnis erreicht hat. So kann die Reinigungsvorrichtung, insbesondere die Steuerung, beispielsweise eingerichtet sein, um den Desinfektionsschritt so lange durchzuführen, bis das tatsächliche Desinfektionsergebnis das mindestens eine vorgegebene Desinfektionsergebnis erreicht.

Die Reinigungsvorrichtung kann insbesondere mindestens einen Temperatursensor umfassen, welcher eine Temperatur innerhalb der Reinigungskammer erfasst, beispielsweise mindestens eine Temperatur, mit welcher der Behälter beaufschlagt wird. Der mindestens eine Temperatursensor kann beispielsweise wiederum einen elektrischen Temperatursensor und/oder einen optischen Temperatursensor umfassen, beispielsweise mindestens einen temperaturabhängigen Widerstand. Auch andere Arten von Sensoren sind jedoch grundsätzlich möglich. Der mindestens eine Temperatursensor kann insbesondere mit der mindestens einen Steuerung verbunden sein, beispielsweise zur Bestimmung des tatsächlichen Desinfektionsergebnisses.

Zur Abfrage des Füllstands in dem Tank des Dampferzeugers weist die Reinigungsvorrichtung mindestens einen Niveausensor zur Erfassung eines Flüssigkeitsniveaus in dem Tank des Dampferzeugers auf. Der Niveausensor kann beispielsweise im Tank des Dampferzeugers vorgesehen sein.

Unter einem Niveausensor kann dabei allgemein im Rahmen der vorliegenden Erfindung ein beliebiger Sensor verstanden werden, welcher eingerichtet ist, um ein Flüssigkeitsniveau zu messen und/oder zu erfassen. Der Sensor kann eingerichtet sein, um ein Erreichen mindestens eines bestimmten, vorgegebenen oder vorgebbaren Zielniveaus zu detektieren und/oder kann eingerichtet sein, um ein Messergebnis auszugeben, welches ein aktuelles Niveau charakterisiert.

Der mindestens eine Niveausensor kann dabei grundsätzlich auf beliebige Messprinzipien zurückgreifen, welche zur Messung eines Flüssigkeitsniveaus geeignet sind. Beispielsweise können dabei elektrische und/oder mechanische und/oder hydraulische und/oder optische Messmethoden eingesetzt werden. So kann der mindestens eine Niveausensor insbesondere ausgewählt sein aus der Gruppe bestehend aus: einem elektrischen Niveausensor, beispielsweise mittels mindestens zweier Elektroden zur Leitwertmessung; einem Drucksensor zur Erfassung eines durch eine Flüssigkeitssäule erzeugten Drucks; einem mechanischen Niveausensor, beispielsweise mittels mindestens eines Schwimmers und/oder Schalters; einem optischen Niveausensor, beispielsweise mittels mindestens einer Lichtschranke. Beispielsweise kann die Niveaumessung fest, beispielsweise mit einer Leitwertmessung mit festen Elektroden, und/oder stufenlos, beispielsweise mit Drucksensoren, durchgeführt werden.

Allgemein kann die Niveaumessung mittels des Niveausensors stufenweise und/oder stufenlos erfolgen. So kann der Niveausensor beispielsweise mindestens einen Stufensensor umfassen, wobei der Stufensensor eingerichtet sein kann, um ein Erreichen mindestens eines vorgegebenen Flüssigkeitsniveaus zu detektieren.

Die Steuerung kann insbesondere mit dem mindestens einen Niveausensor verbunden sein, beispielsweise mittels mindestens einer drahtlosen Verbindung und/oder mindestens einer drahtgebundenen Verbindung.

Die Steuerung kann insbesondere eingerichtet sein, um der ausgewählten Flüssigkeitsmenge ein Zielniveau zuzuordnen und um das Befüllen des Tanks des Dampferzeugers bei Erreichen des Zielniveaus zu beenden.

Der Dampferzeuger kann beispielsweise mindestens ein Heizelement aufweisen, beispielsweise mindestens eine Heizspirale. Beispielsweise kann dieses mindestens eine Heizelement in dem Tank des Dampferzeugers angeordnet sein.

Beispielsweise kann ein Mindestniveau an Flüssigkeit in dem Dampferzeuger dadurch vorgegeben sein, dass vorzugsweise das Heizelement vollständig mit Flüssigkeit bedeckt sein muss. Dieses Mindestniveau kann beispielsweise einer Mindestmenge an Flüssigkeit in dem Tank entsprechen. Die Steuerung kann beispielsweise derart eingerichtet sein, dass sichergestellt ist, dass im Tank des Dampferzeugers stets Flüssigkeit bis hin zu dem Mindestniveau vorliegt. Durch die Verwendung mindestens eines Niveausensors kann beispielsweise eine aktuelle Füllmenge in dem Tank abgefragt werden und beispielsweise mit der Mindestfüllmenge verglichen werden. So kann die Mindestfüllmenge im Tank des Dampferzeugers abgefragt werden, sodass beispielsweise ein Schutz für ein Heizelement des Dampferzeugers gegen Trockenheizen besteht.

Die Reinigungsvorrichtung ist vorzugsweise eingerichtet, um die für den Desinfektionsschritt benötigte Füllmenge im Dampferzeuger zu optimieren. Zu diesem Zweck wird vorzugsweise, wie oben ausgeführt, das tatsächliche Desinfektionsergebnis durch die Erfassung eines zeitlichen Temperaturverlaufs in der Reinigungskammer überwacht. Die Heißdampfzufuhr kann beispielsweise so lange andauern, bis mindestens das vorgegebene Desinfektionsergebnis erreicht ist.

Die benötigte Heißdampfmenge ist in der Regel von verschiedenen Umständen, wie beispielsweise von der Beschaffenheit des zu desinfizierenden Behälters, von Außentemperatur und Außenluftdruck, abhängig. Die Reinigungsvorrichtung, insbesondere die Steuerung, ist vorzugsweise eingerichtet, um ein iteratives Lernprogramm zur Anpassung der Flüssigkeitsmenge durchzuführen, wobei das tatsächliche Desinfektionsergebnis nach einem ersten Desinfektionsschritt mit einer während dieses ersten Desinfektionsschritts verbrauchten Flüssigkeitsmenge im Tank des Dampferzeugers verglichen wird und wobei für mindestens einen nachfolgenden zweiten Desinfektionsschritt die Flüssigkeitsmenge, mit welcher der Tank des Dampferzeugers befüllt wird, angepasst wird.

Bei diesem iterativen Lernprogramm wird vorzugsweise nach der Durchführung eines Desinfektionsschritts die tatsächlich verbrauchte Flüssigkeitsmenge für ein vorgegebenes Desinfektionsergebnis mit der von der Steuerung für dieses Desinfektionsergebnis vorgegebenen Füllmenge verglichen. Bei der nächsten Durchführung eines Desinfektionsschritts wird von der Steuerung die Füllmenge entsprechend angepasst. Wird beispielsweise festgestellt, dass nach Erreichen des vorgegebenen Desinfektionsergebnisses noch Flüssigkeit oberhalb eines Mindestniveaus im Dampferzeuger vorhanden ist, so kann beispielsweise für den nächsten Desinfektionsschritt mit gleichem vorgegebenen Desinfektionsergebnis die Befüllung des Dampferzeugers entsprechend reduziert werden. Wird hingegen festgestellt, dass zu wenig Flüssigkeit bei Erreichung des vorgegebenen Desinfektionsergebnisses im Dampferzeuger vorhanden war, so kann im nachfolgenden Desinfektionsschritt die Befüllung entsprechend erhöht werden.

Durch eine bedarfsgerechte Befüllung des Tanks des Dampferzeugers kann die Programmlaufzeit verkürzt werden. In einem Füllvorgang des Tanks des Dampferzeugers wird beispielsweise bei einer bedarfsgerechten Füllung nur so viel Flüssigkeit wie benötigt in den Tank gefüllt. So kann ein schneller Füllvorgang und eine kurze Aufheizzeit erreicht werden. Weiter kann auch der Verbrauch von chemischem Dosiermittel, welches mit der Flüssigkeitsmenge im Tank des Dampferzeugers korreliert, verringert werden.

Für einen Hersteller ergibt sich durch die vorgeschlagene erfindungsgemäße Ausgestaltung der Reinigungsvorrichtung nur ein geringer zusätzlicher Aufwand zum Stand der Technik. Beispielsweise werden, bei Verwendung eines ruhenden Niveausensors, in der Regel keine zusätzlichen beweglichen Bauteile benötigt.

Der oben beschriebene optionale Überlauf kann vorzugsweise oberhalb mindestens eines vorgegebenen oder vorgebbaren Niveaus im Fluidtank angeordnet sein, welches die benötigte Flüssigkeitsmenge für den mindestens einen Waschschritt angibt und welches beispielsweise als Waschniveau bezeichnet werden kann. So können beispielsweise in dem Fluidtank ein oder mehrere Waschniveaus vorgegeben oder vorgebbar sein oder auf andere Weise wählbar sein, welche jeweils einer für einen Waschschritt benötigten Menge an Reinigungsfluid entsprechen. Weiterhin kann mindestens ein Füllniveau im Fluidtank vorgesehen sein, welches während des Befüllschritts eingenommen wird, um den Tank des Dampferzeugers zu befüllen und welches beispielsweise als Desinfektions-Füllniveau bezeichnet werden kann. Das Desinfektions-Füllniveau oder, falls mehrere Desinfektions-Füllniveaus vorgegeben sind, das unterste Desinfektions-Füllniveau, kann mindestens auf gleicher Höhe oder höher angeordnet sein wie das Waschniveau oder, falls mehrere Waschniveaus vorgesehen sind, als das oberste Waschniveau. Das Desinfektions-Füllniveau kann beispielsweise durch die Überlaufkante des Überlaufs vorgegeben sein.

In Vorbereitung mindestens eines Waschschritts kann der Fluidtank bis zu dem mindestens einen Waschniveau befüllt werden, ohne dass Reinigungsfluid über den Überlauf in den Tank des Dampferzeugers strömt. So können beispielsweise ein oder mehrere Waschschritte ohne eine Befüllung des Tanks des Dampferzeugers erfolgen oder in einem Zustand, in welchem der Tank des Dampferzeugers bereits befüllt ist. Mittels der vorgeschlagenen Reinigungsvorrichtung lässt sich also eine hohe Flexibilität hinsichtlich einer Programmgestaltung des Reinigungsprogramms erzielen. Dieser Füllvorgang kann beispielsweise als Wasch-Befüllschritt bezeichnet werden. Der Wasch-Befüllschritt kann einfach oder auch mehrfach durchgeführt werden.

Zur Vorbereitung des mindestens einen Desinfektionsschritts kann mindestens ein Befüllschritt durchgeführt werden, bei welchem der Fluidtank bis zu dem Füllniveau befüllt wird und in welchem Reinigungsfluid oder eine Komponente desselben, beispielsweise Wasser, aus dem Fluidtank in den Tank des Dampferzeugers strömt, beispielsweise über den Überlauf. Dieser Befüllschritt kann auch als Desinfektions-Befüllschritt bezeichnet werden und kann einfach oder auch mehrfach durchgeführt werden.

Der Wasch-Befüllschritt kann mindestens einmal vor dem oder vor dem ersten Desinfektions-Befüllschritt durchgeführt werden. In diesem Fall schließt sich vorzugsweise an die Durchführung des Waschschritts der mindestens eine Desinfektions-Befüllschritt an. Alternativ kann der Desinfektions-Befüllschritt jedoch auch mindestens einmal vor oder auch während der ersten Durchführung des Wasch-Befüllschritts durchgeführt werden. Der Wasch-Befüllschritt und der Desinfektions-Befüllschritt können also ganz oder vollständig gleichzeitig durchgeführt werden, oder der Desinfektions-Befüllschritt kann ein Bestandteil des Wasch-Befüllschritts sein. So kann beispielsweise der Fluidtank, wie oben ausgeführt, bis zum Desinfektions-Füllniveau gefüllt werden, und es kann eine Befüllung des Tanks des Dampferzeugers bis zum gewünschten Niveau erfolgen. Dadurch ist vorzugsweise gleichzeitig auch der Fluidtank gefüllt, in diesem Fall bis zum Desinfektions-Füllniveau, welches dann beispielsweise dem obersten Waschniveau im Fluidtank entsprechen kann. Verschiedene andere Ausgestaltungen sind denkbar.

Der Fluidtank kann also allgemein mindestens ein Waschniveau und mindestens ein Desinfektions-Füllniveau aufweisen.

Weiterhin kann die Reinigungsvorrichtung, wie oben ausgeführt, beispielsweise eingerichtet sein, ein gewünschtes Desinfektionsergebnis für eine Beaufschlagung mit Thermoäquivalenten mittels der Steuerung auszuwählen und/oder vorzugeben, beispielsweise A0 = 60 oder eine höhere Desinfektionsleistung, beispielsweise A0 = 600 oder beispielsweise A0 = 3000. Die Steuerung wählt vorzugsweise eine Flüssigkeitsmenge für die benötigte Menge an Heißdampf für das ausgewählte Desinfektionsergebnis aus. In dem Befüllschritt wird dann vorzugsweise der Tank des Dampferzeugers mit dieser Flüssigkeitsmenge befüllt, beispielsweise über den Überlauf des Fluidtanks. Der Tank des Dampferzeugers kann dazu vorzugsweise unterhalb der Überlaufkante des Überlaufs angeordnet sein. Beispielsweise kann für ein Desinfektionsergebnis A0 = 60 der Füllstand typischerweise ca. 0,4 l Wasser im Tank des Dampferzeugers betragen. Beispielsweise mit mindestens einem mit der Steuerung verbundenen Niveausensor kann der Füllstand im Tank des Dampferzeugers erfasst werden. Die Steuerung kann vorzugsweise den Befüllschritt beenden, wenn der von der Steuerung für das vorgegebene Desinfektionsergebnis ausgewählte Füllstand erreicht ist. Beispielsweise kann so bei einem gewünschten Desinfektionsergebnis oder Desinfektionswert A0 = 60 nur so wenig Wasser in den Dampferzeuger und dort insbesondere dessen Tank gefüllt werden, dass die dafür erforderliche Dampfmenge erzeugt werden kann.

In einem zweiten Aspekt der Erfindung wird, wie oben ausgeführt, ein Verfahren zur Reinigung eines Behälters für menschliche Ausscheidungen vorgeschlagen. Dabei wird eine Reinigungsvorrichtung gemäß einer oder mehreren der oben beschriebenen Ausgestaltungen verwendet. Dementsprechend kann für mögliche Ausgestaltungen der Verfahrens auf mögliche Ausgestaltungen der Reinigungsvorrichtung verwiesen werden.

Bei dem Verfahren wird mindestens ein Reinigungsprogramm durchgeführt. Das Reinigungsprogramm umfasst mindestens einen Waschschritt und mindestens einen Desinfektionsschritt, wobei in dem Waschschritt der Behälter in mindestens einer Reinigungskammer mit mindestens einem Reinigungsfluid aus mindestens einem Fluidtank beaufschlagt wird und wobei in dem Desinfektionsschritt der Behälter mit Heißdampf aus einem Dampferzeuger beaufschlagt wird. Für den Desinfektionsschritt wird aus einer Mehrzahl möglicher Flüssigkeitsmengen eine Flüssigkeitsmenge ausgewählt, und ein Tank des Dampferzeugers wird vor Durchführung des Desinfektionsschritts mit der Flüssigkeitsmenge befüllt wird. Dabei wird mindestens einen Niveausensor zur Erfassung eines Flüssigkeitsniveaus in dem Tank des Dampferzeugers verwendet.

### Kurze Beschreibung der Figuren

Weitere mögliche Einzelheiten und Merkmale der Erfindung ergeben sich aus der nachfolgenden Beschreibung bevorzugter Ausführungsbeispiele. Die dargestellten Merkmale können dabei einzeln oder in Kombination realisiert werden, wie der Fachmann erkennen wird. Die Erfindung ist nicht durch die Ausführungsbeispiele beschränkt. Die Ausführungsbeispiele sind schematisch in den Figuren dargestellt. Gleiche Bezugsziffern in den Figuren bezeichnen dabei gleiche oder funktionsgleiche oder hinsichtlich ihrer Funktionen einander entsprechende Elemente.

Es zeigen:
- Figur 1: ein erfindungsgemäße Reinigungsvorrichtung mit einem Fluidtank und einem Dampferzeuger; und
- Figur 2: den Fluidtank mit integriertem Dampferzeuger.

### Beschreibung der Ausführungsbeispiele

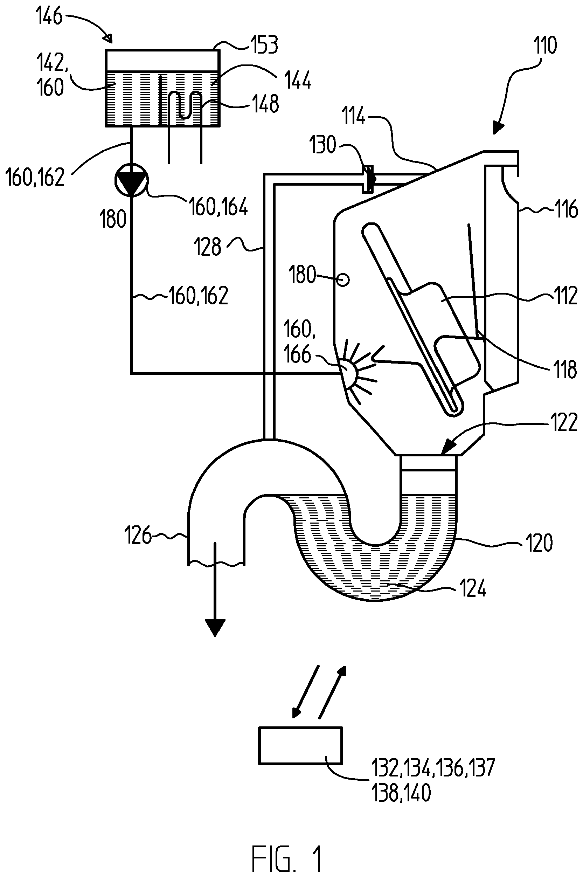

Ein Ausführungsbeispiel einer erfindungsgemäßen Reinigungsvorrichtung 110 zur Reinigung eines Behälters 112 für menschliche Ausscheidungen ist in Figur 1 dargestellt. Die Reinigungsvorrichtung 110 weist eine Reinigungskammer 114 auf. Der Behälter 112 kann beispielsweise durch eine Tür 116, beispielsweise wie in Figur 1 ausgestaltet als Frontklappe, oder auch auf andere Weise ausgestaltet, in die Reinigungskammer 114 eingebracht werden. Das Öffnen und/oder Schließen der Tür 116 kann automatisch, beispielsweise durch mindestens einen in der Figur 1 nicht dargestellten Antrieb und/oder manuell erfolgen. In der Reinigungskammer 114 kann der Behälter 112 durch eine Halterung 118, beispielsweise ein Drahtgestänge und/oder einen Drahtkorb oder eine andere Art von Halterung 118, fixiert werden.

Am Boden der Reinigungskammer 114 ist ein Abfluss 120 mit einer entsprechend großen Abflussöffnung 122 angeordnet, um den Behälter 112 zu entleeren und größere Abfallmengen zu entsorgen. Der Abfluss 120 ist vorzugsweise mit einem Geruchsverschluss 124 versehen. Der Geruchsverschluss 124 kann beispielsweise als Siphonbogen ausgestaltet sein und ist vorzugsweise mit einem oder mehreren Abflussrohren 126 verbunden.

Optional kann mindestens ein Bypass 128 vorgesehen sein. In diesem Bypass 128 kann optional mindestens ein Ventil 130, beispielsweise ein Absperrventil und/oder ein Rückschlagventil, vorgesehen sein, beispielsweise um ein Rückströmen von Gasen und/oder Flüssigkeit aus dem mindestens einen Abflussrohr 126 in die Reinigungskammer 114 zu verhindern. Mit dem Bypass 128 kann beispielsweise Dampf und/oder Gase aus der Reinigungskammer 114 unter Umgehung des Geruchsverschlusses 124 direkt in den Abfluss 120 und/oder das Abflussrohr 126 zwangsverdrängt werden, beispielsweise über mindestens einen in der Figur 1 nicht dargestellten Druckstutzen zur Reinigungskammer 114, über welchen beispielsweise Luft in die Reinigungskammer 114 eingepresst werden kann, um den Behälter 112 zu kühlen und /oder Dampf in den Abfluss 120 zu verdrängen.

Die Reinigungsvorrichtung 110 weist mindestens eine Steuerung 132 auf, welche in Figur 1 nur schematisch dargestellt ist. Die Steuerung 132 kann beispielsweise mindestens eine Datenverarbeitungsvorrichtung 134 und optional mindestens einen Datenspeicher 136, beispielsweise einen flüchtigen und/oder einen nicht flüchtigen Datenspeicher, umfassen. Zudem kann die Steuerung 132 beispielsweise zur Maschine-Mensch-Kommunikation und/oder zur Maschine-Maschine-Kommunikation eingerichtet sein und kann optional eine oder mehrere Schnittstellen 137 aufweisen. Die mindestens eine Schnittstelle 137 kann beispielsweise ein oder mehrere Bedienelemente 138 aufweisen, beispielsweise ausgewählt aus der Gruppe bestehend aus Schaltern, Tastaturen, Bediendisplays und Anzeigevorrichtungen, insbesondere ein oder mehrere Displays und/oder ein oder mehrere optische und/oder akustische Anzeigeelemente 140.

Die Reinigungsvorrichtung 110 ist weiter eingerichtet, um mittels der Steuerung 132 mindestens ein Reinigungsprogramm durchzuführen. Dabei können ein oder mehrere Reinigungsprogramme vorgegeben sein und/oder vorgebbar sein. Vor Beginn des Reinigungsprogramms kann beispielsweise ein Reinigungsprogramm ausgewählt werden. Der Start des Reinigungsprogramms kann automatisch oder manuell erfolgen. Beispielsweise kann der Start durch Betätigen eines Bedienelements 138, durch Betätigung eines Knopfes oder Schalters, beispielsweise eines Fußpedals, und/oder auch durch Schließen der Tür 116 erfolgen.

Das Reinigungsprogramm umfasst mindestens einen Wasch- und mindestens einen Desinfektionsschritt. Während des Reinigungsprogramms wird der Behälter 112 mit Reinigungsfluid beaufschlagt. Zu diesem Zweck umfasst die Reinigungsvorrichtung 110 mindestens einen Fluidtank 142 und einen Dampferzeuger 144.

Zur Beaufschlagung des Behälters 112 mit dem Reinigungsfluid weist die Reinigungsvorrichtung 110 mindestens eine Fluidvorrichtung 160 auf. Die Fluidvorrichtung 160 kann beispielsweise, wie in Figur 1 dargestellt, den mindestens einen Fluidtank 142 sowie mindestens eine Rohrleitung 162 und optional mindestens eine Pumpe 164, auch als Waschpumpe bezeichnet, umfassen. Weiterhin kann die Fluidvorrichtung 160 beispielsweise mindestens eine Düse 166 aufweisen, mittels derer Reinigungsfluid auf den Behälter 112 aufgebracht werden kann, beispielsweise durch Aufsprühen, Aufstrahlen, Auftropfen oder auf andere Weise.

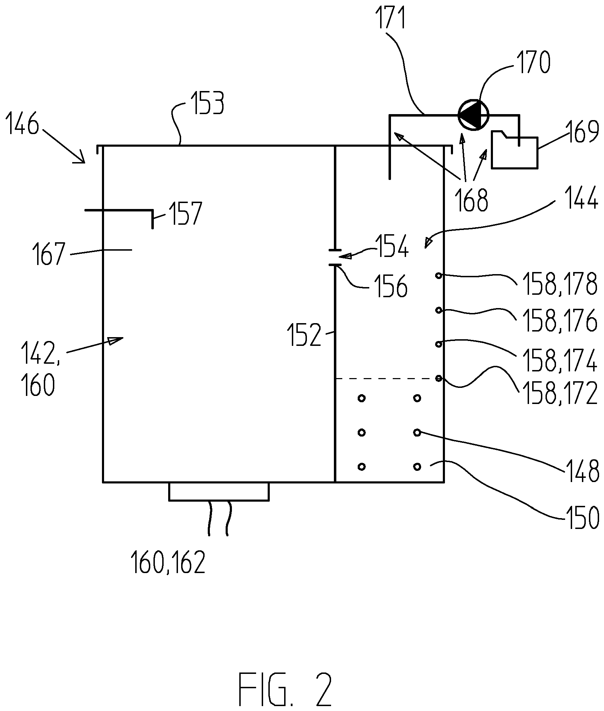

Eine mögliche Ausführungsform des Fluidtanks 142 und des Dampferzeugers 144 ist in Figur 2 dargestellt. In dieser Ausführungsform sind der Fluidtank 142 und der Dampferzeuger 144 in einer gemeinsamen Fluideinheit 146 integriert. Der Dampferzeuger 144 kann zur Dampferzeugung mit mindestens einem Heizelement 148 und mindestens einem Tank 150 eingerichtet sein. Der Fluidtank 142 und der Tank 150 sind vorzugsweise fluidisch verbunden. Beispielsweise kann der Fluidtank 142 durch eine Trennwand 152 von dem Dampferzeuger 144 getrennt sein, wobei die Trennwand 152 vorzugsweise nicht bis zu einem gemeinsamen Deckel 153 der Fluideinheit 146 reicht, sondern vorzugsweise eine Verbindung zwischen dem Fluidtanks 142 und dem Tank 150 des Dampferzeugers 144 ermöglicht. Der Fluidtank 142 ist beispielsweise mit mindestens einem Überlauf 154 mit einer Überlaufkante 156 eingerichtet, um den Tank 150 des Dampferzeugers 144 über den Überlauf 154 zu befüllen. Dazu ist der Tank 150 des Dampferzeugers 144 vorzugsweise unterhalb der Überlaufkante 156 angeordnet. Es wird darauf hingewiesen, dass diese Art der fluidischen Verbindung zwischen dem Fluidtank 142 und dem Tank 150 lediglich eine mögliche Ausgestaltung ist und das andere Ausgestaltungen fluidischer Verbindungen alternativ oder zusätzlich realisierbar sind, wie beispielsweise Verbindungen durch Rohrleitungen, Öffnungen in der Trennwand 152 oder andere Arten fluidischer Verbindungen.

Der Fluidtank 142 wird während des Reinigungsprogramms vorzugsweise mindestens einmal mit Reinigungsfluid oder einen Teil des Reinigungsfluids befüllt, beispielsweise mit Wasser, vorzugsweise Frischwasser. Zu diesem Zweck kann der Fluidtank 142 beispielsweise mindestens einen Frischwasserzulauf 157 aufweisen. Der Frischwasserzulauf 157 kann beispielsweise mit einem bauseitigen Frischwasseranschluss verbunden sein und/oder mit einer Wasseraufbereitungsvorrichtung, beispielsweise zum Reinigen und/oder Erwärmen von Wasser. Weiterhin kann die Fluideinheit 146 eine oder mehrere Dosiervorrichtungen aufweisen, welche in Figur 2 nicht dargestellt sind und mittels derer beispielsweise ein oder mehrere Zusatzstoffe in den Fluidtank 142 eindosiert werden können. Diese mindestens eine Dosiervorrichtung kann beispielsweise mindestens einen Tank für Zusatzstoffe und mindestens eine Rohrleitung umfassen sowie beispielsweise mindestens ein Dosierelement wie beispielsweise mindestens eine Düse und/oder mindestens ein Ventil und/oder mindestens eine Pumpe. Beispielsweise können auf diese Weise dem Reinigungsfluid Desinfektionsmittel und/oder Reinigerkonzentrate und/oder andere Zusatzstoffe beigemischt werden, wie beispielsweise Zusatzstoffe zur Verhinderung von Kalkablagerungen und/oder Klarspüler.

Der Fluidtank 142 wird zur Befüllung des Dampferzeugers 144 vorzugsweise bis zum Überlauf 154 mit Reinigungsfluid oder, was im Rahmen der Befüllung gleichbedeutend sein soll, einer Komponente desselben, beispielsweise Wasser, gefüllt, so dass das Reinigungsfluid in den Tank 150 des Dampferzeugers 144 strömt.

Der Dampferzeuger 144 kann vorzugsweise mindestens einen Niveausensor 158 aufweisen. Vorzugsweise kann dieser Niveausensor 158 ein fester oder ruhender Niveausensor sein, beispielsweise zur Durchführung einer Grenzmessung mit Füllstandsgrenzschalter und/oder zur Durchführung einer Leitwertmessung mit festen Elektroden. Alternativ oder zusätzlich kann der Flüssigkeitsstand auch kontinuierlich bestimmt werden, beispielsweise mittels einem oder mehreren Drucksensoren. Auch ein beweglicher Niveausensor ist generell möglich.

Im dargestellten Ausführungsbeispiel gemäß Figur 2 sind exemplarisch vier verschiedene feste Niveausensoren 158 dargestellt, welche jeweils ein Erreichen eines vorgegebenen Füllstandes in dem Tank 150 detektieren können. So kann beispielsweise ein Mindestniveausensor 172 vorgesehen sein, welcher detektiert, ob eine Mindestfüllmenge in dem Tank 150 vorliegt, welche zur Bedeckung des Heizelements 148 ausreicht. Auf diese Weise kann beispielsweise ein Trockenheizen verhindert werden. Weiterhin können Niveausensoren 158 vorgesehen sein, welche jeweils beispielsweise einem vorgegebenen Desinfektionsergebnis entsprechen. Beispielsweise kann ein Niveausensor 174 für eine Beaufschlagung mit Thermoäquivalenten A0 = 60 vorliegen, ein weiterer Niveausensor 176 für eine Beaufschlagung mit Thermoäquivalenten A0 = 600 und ein weiterer Niveausensor 178 für eine Beaufschlagung mit der Thermoäquivalenten A0 = 3000. Diese Niveaus können beispielsweise empirisch bestimmt werden oder auch beispielsweise iterativ mittels eines entsprechenden iterativen Lernprogramms. Beispielsweise kann eine Füllmenge für A0 = 60 ca. 0,4 l Wasser betragen. Der mindestens eine Niveausensor 158 kann beispielsweise über mindestens eine Verbindung, welche drahtlos oder auch drahtgebundenen ausgestaltet sein kann, mit der mindestens einen Steuerung 132 verbunden sein, beispielsweise um Informationen über einen Füllstand und/oder ein erreichtes Flüssigkeitsniveau in dem Tank 150 an die Steuerung 132 zu übermitteln.

Die Steuerung 132 ist vorzugsweise so eingerichtet, dass der Benutzer ein vorgebbares Desinfektionsergebnis, und damit eine Füllmenge für den Tank 150 des Dampferzeugers 144, auswählen kann. Während des Befüllvorgangs misst der mindestens eine Niveausensor 158 die Füllmenge und/oder erfasst, ob jeweils ein bestimmtes Niveau erreicht ist. Sobald die Flüssigkeitsmenge für ein vorgegebenes Desinfektionsergebnis erreicht ist, stoppt die Steuerung 132 vorzugsweise die Befüllung des Fluidtanks 142, so dass keine Flüssigkeit mehr über den Überlauf 154 in den Tank 150 des Dampferzeugers 144 läuft.

Vor Durchführung des mindestens einen Waschschritts erfolgt vorzugsweise, wie oben ausgeführt, eine Entleerung des Behälters 112, beispielsweise in den Abfluss 120. Dies kann beispielsweise automatisch erfolgen, beispielsweise beim Schließen der Tür 116. In dem mindestens einen Waschschritt wird das Reinigungsfluid aus dem Fluidtank 142 der Fluidvorrichtung 160 zugeführt. Mit der Fluidvorrichtung 160 kann der Behälter 112 einseitig oder von mehreren Seiten mit Reinigungsfluid beaufschlagt werden. Anschließend können ein oder mehrere weitere Waschschritte erfolgen, bei denen der Fluidtank 142 bis zu einem bestimmten Waschniveau 167, welches durch die Menge an Reinigungsfluid für einen Waschgang bestimmt ist, gefüllt wird. Dieses Waschniveau 167 liegt unterhalb des Überlaufs 154, so dass der Dampferzeugertank 150 nicht befüllt wird. Waschschritte können also unabhängig von der Befüllung des Tanks 150 des Dampferzeugers 144 erfolgen.

Weiter sieht das Reinigungsprogramm mindesten einen Desinfektionsschritt vor, bei welchem eine Desinfektion des Behälters 112 durchgeführt wird. Dazu wird in dem Dampferzeuger 144 Heißdampf erzeugt und in die Reinigungskammer 114 geleitet. Dort wird der Behälter 112 mit dem Heißdampf beaufschlagt.

Dem Reinigungsfluid in dem Tank 150 des Dampferzeugers 144 und/oder dem Heißdampf selbst können optional eine oder mehrere Zusatzstoffe beigemischt werden. Zu diesem Zweck kann beispielsweise mindestens eine weitere Dosiervorrichtung 168 für den Dampferzeuger 144 vorgesehen sein. Diese kann beispielsweise wiederum mindestens einen Tank 169 für mindestens einen Zusatzstoff, mindestens eine optionale Pumpe 170 und mindestens eine Rohrleitung 171 umfassen. Beispielsweise können auf diese Weise Desinfektionsmittel und/oder chemische Stoffe beigemischt werden, welche eine Ablagerung von Kalkbelägen verhindern.

Die Reinigungsvorrichtung 110 kann weiterhin, wie in Figur 1 symbolisch dargestellt, mindestens einen Temperatursensor 180 umfassen, beispielsweise mindestens einen in der Reinigungskammer 114 angeordneten Temperatursensor. Der mindestens eine Temperatursensor 180 kann beispielsweise über mindestens eine Verbindung mit der mindestens einen Steuerung 132 verbunden sein, wobei die Verbindung grundsätzlich drahtlos oder auch drahtgebunden ausgestaltet sein kann. Auf diese Weise können beispielsweise aktuelle Temperaturinformationen aus dem Inneren der Reinigungskammer 114 an die Steuerung 132 übermittelt werden. Die Steuerung 132 kann insbesondere eingerichtet sein, um einen zeitlicher Temperaturverlauf in der Reinigungskammer 114 zu erfassen. Auf diese Weise kann beispielsweise ein tatsächliches oder aktuelles Desinfektionsergebnis erfasst werden und vorzugsweise mit einem vorgegebenen Desinfektionsergebnis verglichen werden. Beispielsweise können auf diese Weise Thermoäquivalente erfasst werden, mit denen der Behälter 112 bereits beaufschlagt wurde, und die Thermoäquivalente können mit vorgegebenen Thermoäquivalenten verglichen werden. Beispielsweise kann die Steuerung 132 derart eingerichtet sein, dass eine Heißdampfzufuhr von der Steuerung 132 erst beendet wird, wenn das von dem Benutzer ausgewählte Desinfektionsergebnis erreicht ist.

Die Steuerung 132 der Reinigungsvorrichtung 110 kann vorzugsweise eingerichtet sein, ein iteratives Lernprogramm durchzuführen. Mit diesem Lernprogramm kann die durch die Steuerung 132 ausgewählte Füllmenge des Tanks 150 des Dampferzeugers 144 für ein vorgegebenes Desinfektionsergebnis optimiert werden. Nach dem abgeschlossenen Desinfektionsschritt vergleicht beispielsweise die Steuerung 132 die von ihr ausgewählte Füllmenge für den Tank 150 des Dampferzeugers 144 für das vom Benutzer gewählte Desinfektionsergebnis mit dem tatsächlichen Verbrauch. So haben beispielsweise die Außentemperatur oder der Außenluftdruck und auch die Beschaffenheit des zu reinigenden Behälters 112 einen Einfluss auf die Dauer des Desinfektionsschritts und die dabei verbrauchte Dampfmenge. In einem späteren Betrieb kann die Steuerung 132 die vorgegebene Füllmenge an den tatsächlichen Verbrauch anpassen.

### Bezugszeichenliste

- 110: Reinigungsvorrichtung
- 112: Behälter
- 114: Reinigungskammer
- 116: Tür
- 118: Halterung
- 120: Abfluss
- 122: Abflussöffnung
- 124: Geruchsverschluss
- 126: Abflussrohr
- 128: Bypass
- 130: Ventil
- 132: Steuerung
- 134: Datenverarbeitungsvorrichtung
- 136: Datenspeicher
- 137: Schnittstelle
- 138: Bedienelemente
- 140: Anzeigeelemente
- 142: Fluidtank
- 144: Dampferzeuger
- 146: Fluideinheit
- 148: Heizelement
- 150: Dampferzeugertank
- 152: Trennwand
- 153: Deckel
- 154: Überlauf
- 156: Überlaufkante
- 157: Frischwasserzulauf
- 158: Niveausensor
- 160: Fluidvorrichtung
- 162: Rohrleitung
- 164: Pumpe
- 166: Düse
- 167: Waschniveau
- 168: Dosiervorrichtung
- 169: Tank
- 170: Pumpe
- 171: Rohrleitung
- 172: Mindestniveausensor
- 174: Niveausensor für A0 = 60
- 176: Niveausensor für A0 = 600
- 178: Niveausensor für A0 = 3000
- 180: Temperatursensor

## Patentansprüche

1. Reinigungsvorrichtung (110) zur Reinigung mindestens eines Behälters (112) für menschliche Ausscheidungen, umfassend mindestens eine Reinigungskammer (114) mit mindestens einer Fluidvorrichtung (160) zur Beaufschlagung des Behälters (112) mit mindestens einem Reinigungsfluid, wobei die Reinigungsvorrichtung (110) mindestens eine Steuerung (132) aufweist, wobei die Steuerung (132) eingerichtet ist, um mindestens ein Reinigungsprogramm durchzuführen, wobei das Reinigungsprogramm mindestens einen Waschschritt und mindestens einen Desinfektionsschritt umfasst, wobei in dem Waschschritt der Behälter (112) mit dem Reinigungsfluid beaufschlagt wird und wobei in dem Desinfektionsschritt der Behälter (112) mit Heißdampf beaufschlagt wird, wobei die Reinigungsvorrichtung (110) mindestens einen Dampferzeuger (144) aufweist, wobei die Steuerung (132) eingerichtet ist, um für den Desinfektionsschritt aus einer Mehrzahl möglicher Flüssigkeitsmengen eine Flüssigkeitsmenge auszuwählen und einen Tank (150) des Dampferzeugers (144) vor Durchführung des Desinfektionsschritts mit der ausgewählten Flüssigkeitsmenge zu befüllen, wobei die Reinigungsvorrichtung (110) mindestens einen Niveausensor (158) zur Erfassung eines Flüssigkeitsniveaus in dem Tank (150) des Dampferzeugers (144) aufweist, wobei der Niveausensor (158) eingerichtet ist, ein Erreichen mindestens eines bestimmten, vorgegebenen oder vorgebbaren Zielniveaus zu detektieren und/oder um ein Messergebnis auszugeben, welches ein aktuelles Niveau charakterisiert, wobei die Reinigungsvorrichtung (110) eingerichtet ist, um den Tank (150) des Dampferzeugers (144) über mindestens einen Überlauf (154) aus dem Fluidtank (142) zu befüllen, wobei die Reinigungsvorrichtung (110) eingerichtet ist um vor dem Waschschritt mindestens einen Befüllschritt durchzuführen, wobei in dem Befüllschritt über den Überlauf (154) die ausgewählte Flüssigkeitsmenge in den Tank (150) des Dampferzeugers (144) gefüllt wird.

2. Reinigungsvorrichtung (110) nach dem vorhergehenden Anspruch, wobei die Steuerung (132) eingerichtet ist, um der ausgewählten Flüssigkeitsmenge ein Zielniveau zuzuordnen und um das Befüllen des Tanks (150) des Dampferzeugers (144) bei Erreichen des Zielniveaus zu beenden.

3. Reinigungsvorrichtung (110) nach einem der vorhergehenden Ansprüche, wobei die Reinigungsvorrichtung (110) weiterhin mindestens einen Fluidtank (142) zur Aufnahme des Reinigungsfluids aufweist, wobei die Reinigungsvorrichtung (110) eingerichtet ist, um den Tank (150) des Dampferzeugers (144) über den Fluidtank (142) zu befüllen.

4. Reinigungsvorrichtung (110) nach dem vorhergehenden Anspruch, wobei der Dampferzeuger (144) und der Fluidtank (142) in einer gemeinsamen Fluideinheit (146) integriert sind.

5. Reinigungsvorrichtung (110) nach dem vorhergehenden Anspruch, wobei der Überlauf (154) eine Überlaufkante (156) aufweist.

6. Reinigungsvorrichtung (110) nach dem vorhergehenden Anspruch, wobei ein Füllstand des Reinigungsfluids in dem Fluidtank (142) für den Waschschritt unterhalb der Überlaufkante (156) oder höchstens auf gleicher Höhe wie die Überlaufkante (156) liegt.

7. Reinigungsvorrichtung (110) nach einem der drei vorhergehenden Ansprüche, wobei der Tank (150) des Dampferzeugers (144) unterhalb des Überlaufs (154) angeordnet ist, wobei die Reinigungsvorrichtung (110) eingerichtet ist um vor dem Waschschritt mindestens einen Befüllschritt durchzuführen, wobei in dem Befüllschritt über den Überlauf (154) die Flüssigkeitsmenge in den Tank (150) des Dampferzeugers (144) gefüllt wird.

8. Reinigungsvorrichtung (110) nach einem der vorhergehenden Ansprüche, wobei die Steuerung (132) eingerichtet ist, um den Waschschritt unabhängig von einer Befüllung des Tanks (150) des Dampferzeugers (144) durchzuführen.

9. Reinigungsvorrichtung (110) nach einem der vorhergehenden Ansprüche, wobei die Steuerung (132) eingerichtet ist, um die Flüssigkeitsmenge entsprechend einem vorgebbaren Desinfektionsergebnis auszuwählen.

10. Reinigungsvorrichtung (110) nach einem der vorhergehenden Ansprüche, wobei die Reinigungsvorrichtung (110), insbesondere die Steuerung (132), eingerichtet ist, um ein tatsächliches Desinfektionsergebnis einer Desinfektion des Behälters (112) während des Desinfektionsschritts zu überwachen.

11. Reinigungsvorrichtung (110) nach dem vorhergehenden Anspruch, wobei die Steuerung (132) eingerichtet ist, um ein iteratives Lernprogramm zur Anpassung der Flüssigkeitsmenge durchzuführen, wobei das tatsächliche Desinfektionsergebnis nach einem ersten Desinfektionsschritt mit einer während dieses ersten Desinfektionsschritts verbrauchten Flüssigkeitsmenge im Tank (150) des Dampferzeugers (144) verglichen wird und wobei für mindestens einen nachfolgenden zweiten Desinfektionsschritt die Flüssigkeitsmenge, mit welcher der Tank (150) des Dampferzeugers (144) befüllt wird, angepasst wird.

12. Verfahren zur Reinigung eines Behälters (112) für menschliche Ausscheidungen, wobei mindestens ein Reinigungsprogramm durchgeführt wird, wobei das Reinigungsprogramm mindestens einen Waschschritt und mindestens einen Desinfektionsschritt umfasst, wobei in dem Waschschritt der Behälter (112) in mindestens einer Reinigungskammer (114) mit mindestens einem Reinigungsfluid aus mindestens einem Fluidtank (142) beaufschlagt wird und wobei in dem Desinfektionsschritt der Behälter (112) mit Heißdampf aus einem Dampferzeuger (144) beaufschlagt wird, wobei für den Desinfektionsschritt aus einer Mehrzahl möglicher Flüssigkeitsmengen eine Flüssigkeitsmenge ausgewählt wird und wobei ein Tank (150) des Dampferzeugers (144) vor Durchführung des Desinfektionsschritts mit der ausgewählten Flüssigkeitsmenge befüllt wird, wobei mindestens ein Niveausensor (158) zur Erfassung eines Flüssigkeitsniveaus in dem Tank (150) des Dampferzeugers (144) verwendet wird, wobei der Niveausensor (158) eingerichtet ist ein Erreichen mindestens eines bestimmten, vorgegebenen oder vorgebbaren Zielniveaus zu detektieren und/oder, um ein Messergebnis auszugeben, welches ein aktuelles Niveau charakterisiert, wobei der Tank (150) des Dampferzeugers (144) über mindestens einen Überlauf (154) aus dem Fluidtank (142) befüllt wird, wobei vor dem Waschschritt mindestens ein Befüllschritt durchgeführt wird, wobei in dem Befüllschritt über den Überlauf (154) die Flüssigkeitsmenge in den Tank (150) des Dampferzeugers (144) gefüllt wird, wobei eine Reinigungsvorrichtung (110) nach einem der vorhergehenden, eine Reinigungsvorrichtung (110) betreffenden, Ansprüche verwendet wird.

## Claims

1. Cleaning device (110) for cleaning at least one container (112) for human excreta, comprising at least one cleaning chamber (114) having at least one fluid device (160) for applying at least one cleaning fluid to the container (112), wherein the cleaning device (110) has at least one controller (132), wherein the controller (132) is configured to carry out at least one cleaning programme, wherein the cleaning programme comprises at least one washing step and at least one disinfection step, wherein, in the washing step, the cleaning fluid is applied to the container (112), and wherein, in the disinfection step, hot steam is applied to the container (112), wherein the cleaning device (110) has at least one steam generator (144), wherein the controller (132) is configured to select for the disinfection step a quantity of liquid from a plurality of possible quantities of liquid and to fill a tank (150) of the steam generator (144) with the selected quantity of liquid prior to the disinfection step being carried out, wherein the cleaning device (110) has at least one level sensor (158) for detecting a liquid level in the tank (150) of the steam generator (144), wherein the level sensor (158) is configured to detect an attainment of at least one determined, predefined or predefinable target level and/or to output a measurement result which characterizes a present level, wherein the cleaning device (110) is configured to fill the tank (150) of the steam generator (144) via at least one overflow (154) from the fluid tank (142), wherein the cleaning device (110) is configured to carry out prior to the washing step at least one filling step, wherein, in the filling step, the selected quantity of liquid is filled into the tank (150) of the steam generator (144) via the overflow (154).

2. Cleaning device (110) according to the preceding claim, wherein the controller (132) is configured to assign a target level to the selected quantity of liquid and to end the filling of the tank (150) of the steam generator (144) upon attainment of the target level.

3. Cleaning device (110) according to either of the preceding claims, wherein the cleaning device (110) furthermore has at least one fluid tank (142) for receiving the cleaning fluid, wherein the cleaning device (110) is configured to fill the tank (150) of the steam generator (144) via the fluid tank (142) .

4. Cleaning device (110) according to the preceding claim, wherein the steam generator (144) and the fluid tank (142) are integrated in a common fluid unit (146).

5. Cleaning device (110) according to the preceding claim, wherein the overflow (154) has an overflow edge (156).

6. Cleaning device (110) according to the preceding claim, wherein a fill level of the cleaning fluid in the fluid tank (142) for the washing step is below the overflow edge (156) or at most at the same height as the overflow edge (156).

7. Cleaning device (110) according to one of the three preceding claims, wherein the tank (150) of the steam generator (144) is arranged below the overflow (154), wherein the cleaning device (110) is configured to carry out prior to the washing step at least one filling step, wherein, in the filling step, the quantity of liquid is filled into the tank (150) of the steam generator (144) via the overflow (154) .

8. Cleaning device (110) according to one of the preceding claims, wherein the controller (132) is configured to carry out the washing step independently of a filling of the tank (150) of the steam generator (144).

9. Cleaning device (110) according to one of the preceding claims, wherein the controller (132) is configured to select the quantity of liquid according to a predefinable disinfection result.

10. Cleaning device (110) according to one of the preceding claims, wherein the cleaning device (110), in particular the controller (132), is configured to monitor an actual disinfection result of a disinfection of the container (112) during the disinfection step.

11. Cleaning device (110) according to the preceding claim, wherein the controller (132) is configured to carry out an iterative learning program for adapting the quantity of liquid, wherein the actual disinfection result after a first disinfection step is compared with a quantity of liquid in the tank (150) of the steam generator (144) that is used during said first disinfection step, and wherein, for at least one subsequent second disinfection step, the quantity of liquid with which the tank (150) of the steam generator (144) is filled is adapted.

12. Method for cleaning a container (112) for human excreta, wherein at least one cleaning programme is carried out, wherein the cleaning programme comprises at least one washing step and at least one disinfection step, wherein, in the washing step, at least one cleaning fluid from at least one fluid tank (142) is applied to the container (112) in at least one cleaning chamber (114), and wherein, in the disinfection step, hot steam from a steam generator (144) is applied to the container (112), wherein a quantity of liquid from a plurality of possible quantities of liquid is selected for the disinfection step, and wherein a tank (150) of the steam generator (144) is filled with the selected quantity of liquid prior to the disinfection step being carried out, wherein at least one level sensor (158) for detecting a liquid level in the tank (150) of the steam generator (144) is used, wherein the level sensor (158) is configured to detect an attainment of at least one determined, predefined or predefinable target level and/or to output a measurement result which characterizes a present level, wherein the tank (150) of the steam generator (144) is filled via at least one overflow (154) from the fluid tank (142), wherein at least one filling step is carried out prior to the washing step, wherein, in the filling step, the quantity of liquid is filled into the tank (150) of the steam generator (144) via the overflow (154), wherein a cleaning device (110) according to one of the preceding claims relating to a cleaning device (110) is used.

## Revendications

1. Dispositif de nettoyage (110) destiné à nettoyer au moins un conteneur (112) pour déchets humains, le dispositif de nettoyage comprenant au moins une chambre de nettoyage (114) pourvue d'au moins un dispositif à fluide (160) destiné à soumettre le conteneur (112) à au moins un fluide de nettoyage, le dispositif de nettoyage (110) comportant au moins une commande (132), la commande (132) étant conçue pour exécuter au moins un programme de nettoyage, le programme de nettoyage comprenant au moins une étape de lavage et au moins une étape de désinfection, le récipient (112) étant soumis, à l'étape de lavage, à l'action du fluide de nettoyage et le récipient (112) étant soumis, à l'étape de désinfection, à de la vapeur surchauffée, le dispositif de nettoyage (110) comportant au moins un générateur de vapeur (144), la commande (132) étant conçue pour sélectionner une quantité de liquide parmi une pluralité de quantités de liquide possibles pour l'étape de désinfection et pour remplir un réservoir (150) du générateur de vapeur (144) avec la quantité de liquide sélectionnée avant d'effectuer l'étape de désinfection, le dispositif de nettoyage (110) comportant au moins un capteur de niveau (158) destiné à détecter un niveau de liquide dans le réservoir (150) du générateur de vapeur (144), le capteur de niveau (158) étant conçu pour détecter qu'au moins un niveau cible déterminé, spécifié ou pouvant être spécifié a été atteint et/ou pour délivrer un résultat de mesure qui caractérise un niveau actuel, le dispositif de nettoyage (110) étant conçu pour remplir le réservoir (150) du générateur de vapeur (144) par le biais d'au moins un trop-plein (154) du réservoir de fluide (142), le dispositif de nettoyage (110) étant conçu pour effectuer au moins une étape de remplissage avant l'étape de lavage, la quantité sélectionnée de liquide étant introduite à l'étape de remplissage dans le réservoir (150) du générateur de vapeur (144) par le biais du trop-plein (154).

2. Dispositif de nettoyage (110) selon la revendication précédente, la commande (132) étant conçue pour associer un niveau cible à la quantité de liquide sélectionnée et pour arrêter le remplissage du réservoir (150) du générateur de vapeur (144) lorsque le niveau cible est atteint.

3. Dispositif de nettoyage (110) selon l'une des revendications précédentes, le dispositif de nettoyage (110) comprenant en outre au moins un réservoir de fluide (142) destiné à recevoir le fluide de nettoyage, le dispositif de nettoyage (110) étant conçu pour remplir le réservoir (150) du générateur de vapeur (144) par le biais du réservoir de fluide (142).

4. Dispositif de nettoyage (110) selon la revendication précédente, le générateur de vapeur (144) et le réservoir de fluide (142) étant intégrés dans une unité de fluide commune (146).

5. Dispositif de nettoyage (110) selon la revendication précédente, le trop-plein (154) comportant un bord de trop-plein (156).

6. Dispositif de nettoyage (110) selon la revendication précédente, un niveau de remplissage du fluide de nettoyage dans le réservoir de fluide (142) pour l'étape de lavage étant situé au-dessous du bord de trop-plein (156) ou au plus à la même hauteur que le bord de trop-plein (156).

7. Dispositif de nettoyage (110) selon l'une des trois revendications précédentes, le réservoir (150) du générateur de vapeur (144) étant disposé au-dessous du trop-plein (154), le dispositif de nettoyage (110) étant conçu pour effectuer au moins une étape de remplissage avant l'étape de lavage, la quantité de liquide étant introduite à l'étape de remplissage dans le réservoir (150) du générateur de vapeur (144) par le biais du trop-plein (154).

8. Dispositif de nettoyage (110) selon l'une des revendications précédentes, la commande (132) étant conçue pour effectuer l'étape de lavage indépendamment d'un remplissage du réservoir (150) du générateur de vapeur (144).

9. Dispositif de nettoyage (110) selon l'une des revendications précédentes, la commande (132) étant conçue pour sélectionner la quantité de liquide conformément à un résultat de désinfection spécifiable.

10. Dispositif de nettoyage (110) selon l'une des revendications précédentes, le dispositif de nettoyage (110), en particulier la commande (132), étant conçu pour surveiller un résultat de désinfection réel d'une désinfection du récipient (112) pendant l'étape de désinfection.

11. Dispositif de nettoyage (110) selon la revendication précédente, la commande (132) étant conçue pour exécuter un programme d'apprentissage itératif destiné à régler la quantité de liquide, le résultat de désinfection réel après une première étape de désinfection étant comparé à une quantité de liquide, consommée pendant cette première étape de désinfection, dans le réservoir (150) du générateur de vapeur (144) et la quantité de liquide avec laquelle le réservoir (150) du générateur de vapeur (144) est rempli étant adaptée pour au moins une deuxième étape de désinfection ultérieure.

12. Procédé de nettoyage d'un conteneur (112) pour déchets humains, au moins un programme de nettoyage étant exécuté, le programme de nettoyage comprenant au moins une étape de lavage et au moins une étape de désinfection, le récipient (112) étant soumis, à l'étape de lavage, dans au moins une chambre de nettoyage (114) à au moins un fluide de nettoyage provenant d'au moins un réservoir de fluide (142) et le récipient (112) étant soumis, à l'étape de désinfection, à de la vapeur surchauffée provenant d'un générateur de vapeur (144), une quantité de liquide étant sélectionnée parmi la pluralité de quantités de liquide possibles pour l'étape de désinfection et un réservoir (150) du générateur de vapeur (144) étant rempli avec la quantité de liquide sélectionnée avant que l'étape de désinfection ne soit effectuée, au moins un capteur de niveau (158) étant utilisé pour détecter un niveau de liquide dans le réservoir (150) du générateur de vapeur (144), le capteur de niveau (158) étant conçu pour détecter qu'au moins un niveau cible déterminé, spécifié ou spécifiable est atteint et/ou pour délivrer un résultat de mesure qui caractérise un niveau actuel, le réservoir (150) du générateur de vapeur (144) étant rempli depuis le réservoir de fluide (142) par le biais d'au moins un trop-plein (154), au moins une étape de remplissage étant effectuée avant l'étape de lavage, la quantité de liquide étant introduite, à l'étape de remplissage, dans le réservoir (150) du générateur de vapeur (144) par le biais du trop-plein (154), un dispositif de nettoyage (110) selon l'une des revendications précédentes concernant un dispositif de nettoyage (110) étant utilisé.
